# EUROPÄISCHE PATENTSCHRIFT

(11) **EP 0 957 095 B1**
(45) Veröffentlichungstag und Bekanntmachung des Hinweises auf die Patenterteilung: **19.02.2003**
(21) Anmeldenummer: 99106021.1
(22) Anmeldetag: 08.11.1995
(51) Int. Cl.: C07D 249/12, C07D 401/06, C07D 405/06, A01N 43/653

(54) **Mikrobizide Triazolyl-Derivate**
Microbicidal triazolyl derivatives
Dérivés triazolyle microbicides

(30) Priorität: 21.11.1994 DE 4441354; 24.07.1995 DE 19526918; 31.07.1995 DE 19528046
(43) Veröffentlichungstag der Anmeldung: 17.11.1999
(62) Teilanmeldung aus: 95938436.3
(73) Patentinhaber: Bayer CropScience AG, 40789 Monheim (DE)
(72) Erfinder: Jautelat, Manfred, Dr., 51399 Burscheid (DE); Tiemann, Ralf, Dr., 51375 Leverkusen (DE); Dutzmann, Stefan, Dr., 40764 Langenfeld (DE); Hänssler, Gerd, Dr., 51381 Leverkusen (DE); Stenzel, Klaus, Dr., 40595 Düsseldorf (DE)

(56) Entgegenhaltungen:
- EP-A- 0 251 086
- EP-A- 0 297 345
- EP-A- 0 461 502
- EP-A- 0 564 810

## Beschreibung

Die vorliegende Erfindung betrifft neue Triazolyl-Derivate, mehrere Verfahren zu deren Herstellung und deren Verwendung als Mikrobizide.

Es ist bereits bekannt geworden, daß zahlreiche Hydroxyethyl-azolyl-Derivate fungizide Eigenschaften besitzen (vgl. EP-A 0 015 756, EP-A 0 040 345, EP-A 0 052 424, EP-A 0 061 835 und EP-A 0 297 345). Die Einsetzbarkeit dieser Stoffe ist jedoch in manchen Fällen nicht immer befriedigend.

Weiterhin sind aus der EP 0 251 086 - A bestimmte fungizid wirksame Mercapto-Triazole bekannt, in denen das zentrale Kohlenstoffatom (hierbei handelt es sich um dasjenige C-Atom, das die Substituenten B und OR² trägt) so wohl mit einem Aralkenyl-Rest als auch mit einem gegebenenfalls substituierten Phenylrest verbunden sein kann. Verbindungen dieses Typs, in denen außer Aralkenyl auch gegebenenfalls substituiertes Cycloalkyl enthalten ist, werden aber nicht erwähnt.

Es wurden nun neue Triazolyl-Derivate der Formel 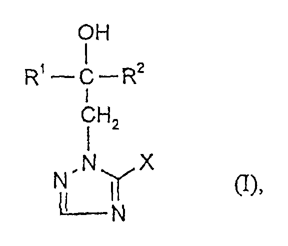 in welcher
- R¹: für geradkettiges oder verzweigtes Alkenyl mit 2 bis 5 Kohlenstoffatomen steht, wobei jeder dieser Reste einfach bis dreifach, gleichartig oder verschieden substituiert sein kann durch Chlor, Brom, Methoxy, Ethoxy, Propoxy, Isopropoxy, Cyclopropyl, Cyclobutyl, Cyclopentyl und/oder Cyclohexyl,
und
- R²: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluorcyclopropyl, 1-Methyl-cyclopropyl, 1-Cyano-Cyclopropyl, Cyclopropyl, 1-Methyl-cyclopentyl oder 1-Ethyl-cyclopentyl steht,
oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
- R¹: für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
- R²: für 1-Methyl-cyclohexyl, Cyclohexyl, 1-Chlor-cyclopropyl, 1-Fluorcyclopropyl, 1-Methyl-cyclopropyl, 1-Cyano-Cyclopropyl, Cyclopropyl, 1-Methyl-cyclopentyl oder 1-Ethyl-cyclopentyl steht,
und
- X: für die Gruppierungen -SH, -SR³, -SO-R³, -SO₂-R³ oder -SO₃H steht, worin
- R³: für Methyl, Ethyl oder Propyl steht, wobei jeder dieser Reste einfach bis dreifach, gleichartig oder verschieden durch Fluor und/oder Chlor substituiert sein kann,
oder
für Allyl, But-2-en-yl oder But-3-enyl steht, wobei jeder dieser Reste einfach bis dreifach, gleichartig oder verschieden substituiert sein kann durch Fluor und/oder Chlor,
oder
für Phenylalkyl mit 1 oder 2 Kohlenstoffatomen im geradkettigen oder verzweigten Alkylteil steht, wobei jeder dieser Reste im Phenylteil einfach oder zweifach substituiert sein kann durch Fluor, Chlor, Brom, Methyl, Ethyl, Tert.-Butyl, Trichlormethyl und/oder Trifluormethyl,
oder
für Phenyl steht, das einfach oder zweifach substituiert sein kann durch Fluor, Chlor, Brom, Methyl, Ethyl, tert.-Butyl, Trichlormethyl und/oder Trifluormethyl,
sowie deren Säureadditions-Salze und Metallsalz-Komplexe gefunden.

Diejenigen erfindungsgemäßen Stoffe, in denen R¹ und R² verschieden sind, enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Weiterhin wurde gefunden, daß man Triazolyl-Derivate der Formel (I) sowie deren Säureadditions-Salze und Metallsalz-Komplexe erhält, wenn man
a) Hydroxyethyl-triazole der Formel 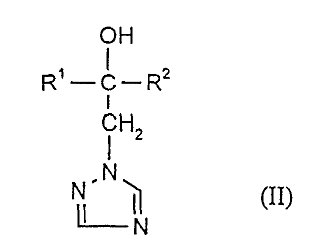 in welcher
   R¹ und R² die oben angegebenen Bedeutungen haben,
   entweder
   α) nacheinander mit starken Basen und Schwefel in Gegenwart eines Verdünnungsmittels umsetzt und dann mit Wasser, gegebenenfalls in Gegenwart einer Säure hydrolysiert,
      oder
   β) mit Schwefel in Gegenwart eines hoch siedenden Verdünnungsmittels umsetzt und dann gegebenenfalls mit Wasser sowie gegebenenfalls mit Säure behandelt,
   und gegebenenfalls die nach den Varianten (α) und (β) entstehenden Verbindungen der Formel 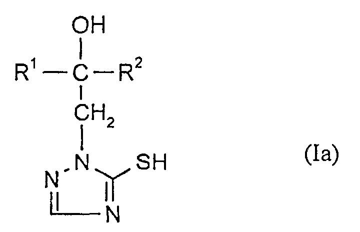 in welcher
   R¹ und R² die oben angegebenen Bedeutungen haben,
   mit Halogen-Verbindungen der Formel

   R⁴-Hal (III)

   in welcher
   - R⁴: für Methyl, Ethyl oder Propyl steht, wobei jeder dieser Reste einfach bis dreifach, gleichartig oder verschieden durch Fluor und/oder Chlor substituiert sein kann
   oder
   für Allyl, But-2-en-yl oder But-3-enyl steht, wobei jeder dieser Reste einfach bis dreifach, gleichartig oder verschieden substituiert sein kann durch Fluor und/oder Chlor,
   oder
   für Phenylalkyl mit 1 oder 2 Kohlenstoffatomen im geradkettigen oder verzweigten Alkylteil steht, wobei jeder dieser Reste im Phenylteil einfach oder zweifach substituiert sein kann durch Fluor, Chlor, Brom, Methyl, Ethyl, tert.-Butyl, Trichlormethyl und/oder Trifluormethyl,
   und
   - Hal: für Chlor, Brom oder Iod steht,
   in Gegenwart eines Säurebindemittels und in Gegenwart eines Verdünnungsmittels umsetzt und gegebenenfalls die dabei entstehenden Verbindungen der Formel 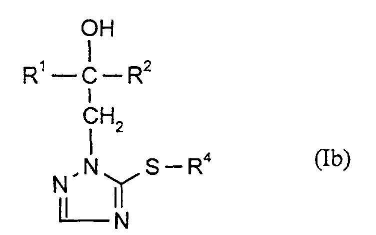 in welcher
   R¹, R² und R⁴ die oben angegebenen Bedeutungen haben,
   mit Oxidationsmitteln in Gegenwart eines Verdünnungsmittels umsetzt,
   oder
b) Hydroxyethyl-triazole der Formel 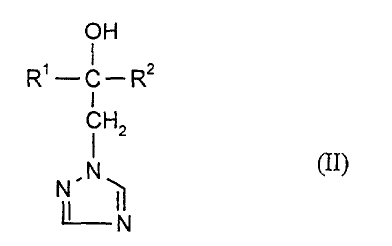 in welcher
   R¹ und R² die oben angegebenen Bedeutungen haben,
   nacheinander mit starken Basen und Diaryl-disulfiden der Formel

   R⁵-S-S-R⁵ (IV)

   in welcher
   - R⁵: für Phenyl steht, das einfach oder zweifach substituiert sein kann durch Fluor, Chlor, Brom, Methyl, ethyl, tert.-Butyl, Trichlormethyl und/oder Trifluormethyl,
   in Gegenwart eines Verdünnungsmittels umsetzt und gegebenenfalls die dabei entstehenden Verbindungen der Formel 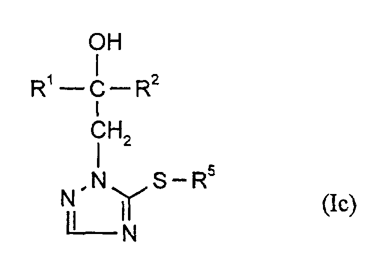 in welcher
   R¹, R² und R⁵ die oben angegebenen Bedeutungen haben,
   mit Oxidationsmitteln in Gegenwart eines Verdünnungsmittels umsetzt,
   oder
c) Triazolyl-Derivate der Formel 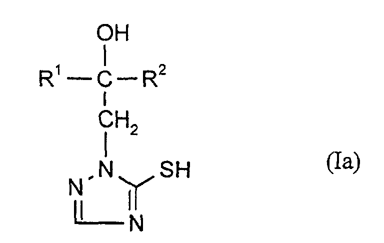 in welcher
   R¹ und R² die oben angegebenen Bedeutungen haben,
   mit Kaliumpermanganat in Gegenwart eines Verdünnungsmittels umsetzt,
und gegebenenfalls anschließend an die erhaltenen Verbindungen der Formel (I) eine Säure oder ein Metallsalz addiert.

Schließlich wurde gefunden, daß die neuen Triazolyl-Derivate der Formel (I) sowie deren Säureadditions-Salze und Metallsalz-Komplexe sehr gute mikrobizide Eigenschaften aufweisen und sich sowohl im Pflanzenschutz als auch im Materialschutz zur Bekämpfung unerwünschter Mikroorganismen, wie Fungi, verwenden lassen.

Überraschenderweise zeigen die erfindungsgemäßen Stoffe eine bessere mikrobizide Wirksamkeit, insbesondere fungizide Wirksamkeit, als die konstitutionell ähnlichsten Verbindungen gleicher Wirkungsrichtung.

Erfindungsgemäße Verbindungen sind auch Additionsprodukte aus Säuren und denjenigen Triazolyl-Derivaten der Formel (I), in denen R¹, R² und X diejenigen Bedeutungen haben, die für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bemsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure, sowie Saccharin und Thiosaccharin.

Erfindungsgemäße Verbindungen sind außerdem Additionsprodukte aus Salzen von Metallen der II. bis IV. Haupt- und der I. und II. sowie IV. bis VIII. Nebengruppe des Periodensystems der Elemente und denjenigen Triazolyl-Derivaten der Formel (I), in denen R¹, R² und X diejenigen Bedeutungen haben, die für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Triazolyl-Derivate der Formel (I), in denen X für eine -SH-Gruppe steht, können in der "Mercapto"-Form der Formel 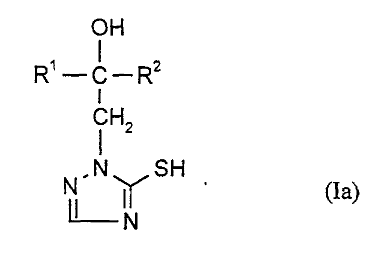 oder in der tautomeren "Thiono"-Form der Formel 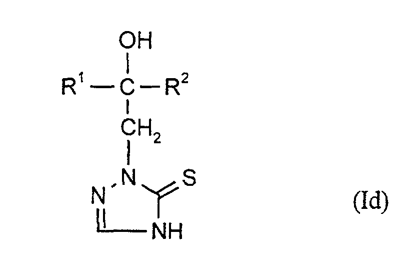 vorliegen. Der Einfachheit halber wird jeweils nur die "Mercapto"-Form aufgeführt.

Als Beispiele für erfindungsgemäße Stoffe sei das in der folgenden Tabelle aufgeführte Triazolyl-Derivat genannt.

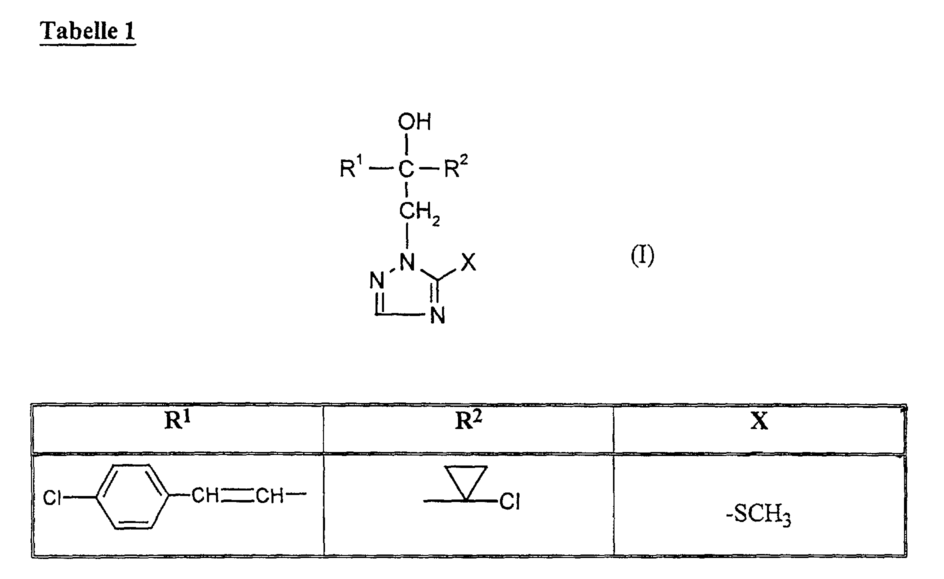

Verwendet man 2-(1-Chlor-cyclopropyl)-1-(2-chlorphenyl)-3-(1,2,4-triazol-1-yl)-propan-2-ol als Ausgangsstoff, n-Butyl-lithium als starke Base und Schwefel-Pulver als Reaktionskomponente, so kann der Verlauf der ersten Stufe des erfindungsgemäßen Verfahrens (a), Variante (α), durch das folgende Formelschema veranschaulicht werden: 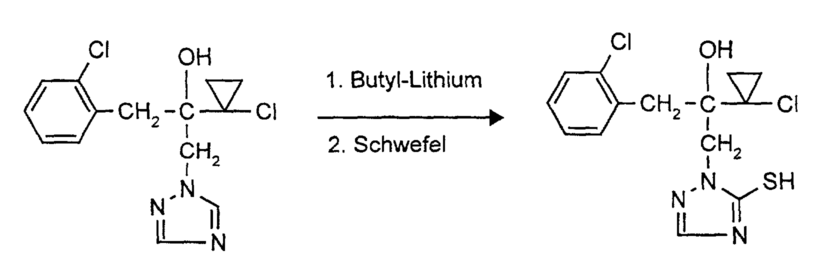 (Es wird darauf hingewiesen, daß die Verbindungen in diesem Formelschema sowie in den sechs folgenden Formelschemata kein Gegenstand der vorliegenden Erfindung sind.)

Verwendet man 2-(1-Chlor-cyclopropyl)-1-(2-Chlor-phenyl)-3-(1,2,4-triazol-1-yl)-propan-2-ol als Ausgangsstoff, Schwefel-Pulver als Reaktionskomponente und N-Methyl-pyrrolidon als Verdünnungsmittel, so kann der Verlauf der ersten Stufe des erfindungsgemäßen Verfahrens (a), Variante (β) durch das folgende Formelschema veranschaulicht werden:

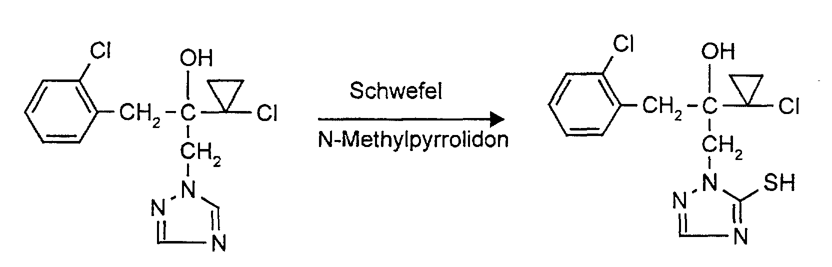

Verwendet man 2-(1-Chlor-cyclopropyl)-1-(2-chlorphenyl)-3-(5-mercapto-1,2,4-triazol-1-yl)-propan-2-ol als Ausgangsstoff und Methyliodid als Reaktionskomponente, so kann der Verlauf der zweiten Stufe des erfindungsgemäßen Verfahrens (a) durch das folgende Formelschema veranschaulicht werden:

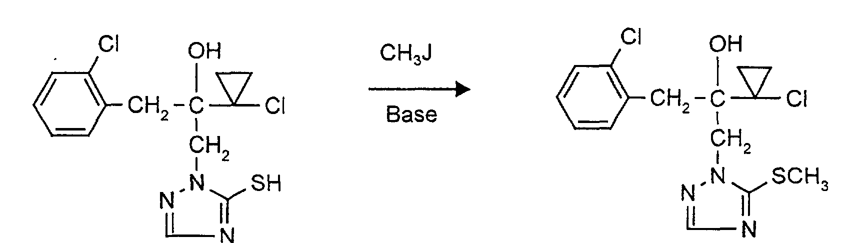

Verwendet man 2-(1-Chlor-cyclopropyl)-1-(2-chlorphenyl)-3-(5-methylthio-1,2,4-triazol-1-yl)-propan-2-ol als Ausgangsstoff und einen Überschuß an Wasserstoffperoxid als Oxidationsmittel, so kann der Verlauf der dritten Stufe des erfindungsgemäßen Verfahrens (a) durch das folgende Formelschema veranschaulicht werden:

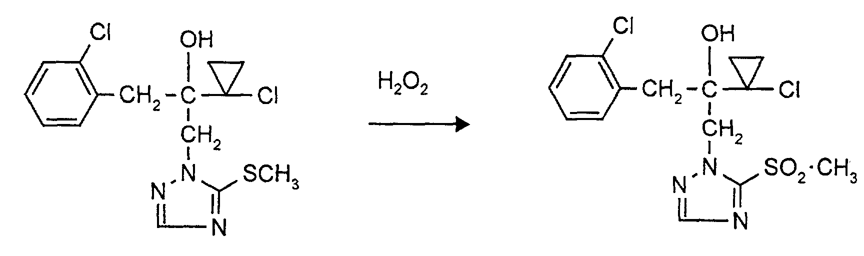

Verwendet man 2-(1-Chlor-cyclopropyl)-1-(2-chlorphenyl)-3-(1,2,4-triazol-1-yl)-propan-2-ol als Ausgangsstoff, n-Butyllithium als starke Base und Diphenyldisulfid als Reaktionskomponente, so kann der Verlauf der ersten Stufe des erfindungsgemäßen Verfahrens (b) durch das folgende Formelschema veranschaulicht werden:

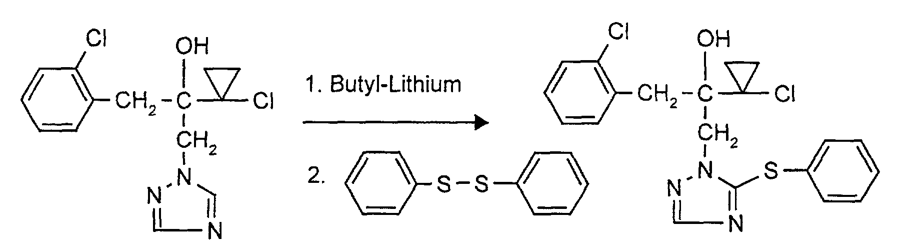

Verwendet man 2-(1-Chlor-cyclopropyl)-1-(2-chlorphenyl)-3-(5-phenylthio-1,2,4-triazol-1-yl)-propan-2-ol und setzt dieses mit einer äquimolaren Menge an Wasserstoffperoxid als Oxidationsmittel um, so kann der Verlauf der zweiten Stufe des erfindungsgemäßen Verfahrens (b) durch das folgende Formelschema veranschaulicht werden:

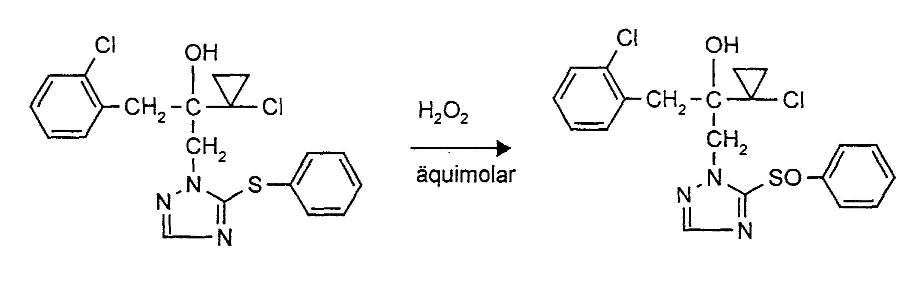

Verwendet man 2-(1-Chlor-cyclopropyl)-1-(2-chlorphenyl)-3-(5-mercapto-1,2,4-triazol-1-yl)-propan-2-ol als Ausgangsstoff und Kaliumpermanganat als Oxidationsmittel, so kann der Verlauf des erfindungsgemäßen Verfahrens (c) durch das folgende Formelschema veranschaulicht werden:

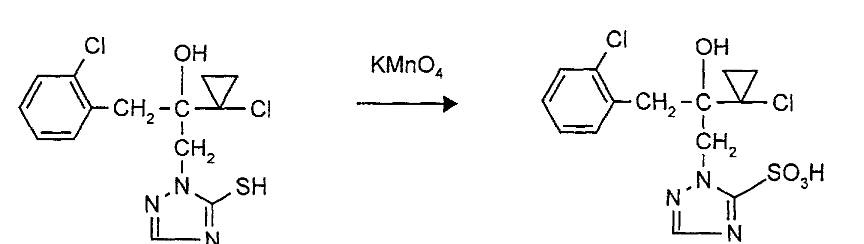

Die bei der Durchführung des erfindungsgemäßen Verfahrens (a) als Ausgangsstoffe benötigten Hydroxyethyl-triazole sind durch die Formel (II) allgemein definiert. In dieser Formel haben R¹ und R² diejenigen Bedeutungen, die bereits im Zusammenhang mit der Beschreibung der erfindungsgemäßen Stoffe der Formel (I) für diese Reste genannt wurden.

Die Hydroxyethyl-triazole der Formel (II) sind bekannt oder lassen sich nach bekannten Methoden herstellen (vgl. EP-A 0 015 756, EP-A 0 040 345, EP-A 0 052 424, EP-A 0 061 835, EP-A 0 297 345 und EP-A 0 470 463).

Als Basen kommen bei der Durchführung der ersten Stufe des erfindungsgemäßen Verfahrens (a), Variante (α), alle für derartige Reaktionen üblichen, starken Alkalimetall-Basen in Betracht. Vorzugsweise verwendbar sind n-Butyl-lithium, Lithiumdiisopropyl-amid, Natriumhydrid, Natriumamid und auch Kalium-tert.-butylat im Gemisch mit Tetramethylethylen-diamin (= TMEDA).

Bei der Durchführung der ersten Stufe des erfindungsgemäßen Verfahrens (a), Variante (α), kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird vorzugsweise in Form von Pulver eingesetzt. Zur Hydrolyse verwendet man bei der Durchführung der ersten Stufe des erfindungsgemäßen Verfahrens (a), Variante (α), Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung der ersten Stufe des erfindungsgemäßen Verfahrens (a), Variante (α), innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung aller Schritte des erfindungsgemäßen Verfahrens (a) arbeitet man im allgemeinen unter Normaldruck. Es ist aber auch möglich, unter erhöhtem oder vermindertem Druck zu arbeiten. So kommt vor allem bei der Durchführung der ersten Stufe des erfindungsgemäßen Verfahrens (a) nach Variante (α) ein Arbeiten unter erhöhtem Druck in Frage.

Bei der Durchführung der ersten Stufe des erfindungsgemäßen Verfahrens (a) nach Variante (α) seht man auf 1 Mol an Hydroxyethyl-triazol der Formel (II) im allgemeinen 2 bis 3 Äquivalente, vorzugsweise 2,0 bis 2,5 Äquivalente, an starker Base und anschließend eine äquivalente Menge oder auch einen Überschuß an Schwefel ein. Die Umsetzung kann unter Schutzgas-atmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Bei der Durchführung der ersten Stufe des erfindungsgemäßen Verfahrens (a) nach Variante (β) kommen als Verdünnungsmittel alle für derartige Umsetzungen üblichen, hoch siedenden organischen Solventien in Betracht. Vorzugsweise verwendbar sind Amide, wie Dimethylformamid und Dimethylacetamid, außerdem heterocyclische Verbindungen, wie N-Methyl-pyrrolidon, und auch Ether, wie Diphenylether.

Schwefel wird auch bei der Durchführung der ersten Stufe des erfindungsgemäßen Verfahrens (a) nach der Variante (β) im allgemeinen in Form von Pulver eingesetzt. Nach der Umsetzung kann gegebenenfalls eine Behandlung mit Wasser sowie gegebenenfalls mit Säure vorgenommen werden. Diese wird so durchgeführt wie die Hydrolyse bei der Durchführung der ersten Stufe des erfindungsgemäßen Verfahrens (a) nach der Variante (α).

Die Reaktionstemperaturen können auch bei der Durchführung der ersten Stufe des erfindungsgemäßen Verfahrens (a) nach Variante (β) innerhalb eines größeren Bereiches variiert werden. Im allgemeinen arbeitet man bei Temperaturen zwischen 150°C und 300°C, vorzugsweise zwischen 180°C und 250°C.

Bei der Durchführung der ersten Stufe des erfindungsgemäßen Verfahrens (a) nach Variante (β) setzt man auf 1 Mol an Hydroxyethyl-triazol der Formel (II) im allgemeinen 1 bis 5 Mol, vorzugsweise 1,5 bis 3 Mol an Schwefel ein. Die Aufarbeitung erfolgt nach üblichen Methoden. Im allgemeinen geht man so vor, daß man das Reaktionsgemisch mit einem in Wasser nur wenig löslichen organischen Solvens extrahiert, die vereinigten organischen Phasen trocknet und einengt und den verbleibenden Rückstand gegebenenfalls nach üblichen Methoden, wie Umkristallisation oder Chromatographie, von eventuell vorhandenen Verunreinigungen befreit.

Die bei der Durchführung der zweiten Stufe des erfindungsgemäßen Verfahrens (a) als Ausgangssubstanzen benötigten Verbindungen der Formel (Ia) sind erfindungsgemäße Stoffe.

Die bei der Durchführung des erfindungsgemäßen Verfahrens (a) in der zweiten Stufe als Reaktionskomponenten benötigten Halogen-Verbindungen sind durch die Formel (III) allgemein definiert.

Die Halogen-Verbindungen der Formel (III) sind bekannt.

Als Säurebindemittel kommen bei der Durchführung der zweiten Stufe des erfindungsgemäßen Verfahrens (a) alle üblichen anorganischen oder organischen Basen in Frage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens (a) alle für derartige Umsetzungen üblichen, inerten organischen Solventien in Betracht. Vorzugsweise verwendbar sind Ether, wie Diethylether, Methyl-tert.-butyl-ether, Ethylenglykol-dimethylether, Tetrahydrofuran und Dioxan, ferner Nitrile, wie Acetonitril, und außerdem stark polare Solventien, wie Dimethylsulfoxid oder Dimethylformamid.

Die Reaktionstemperaturen können bei der Durchführung der zweiten Stufe des erfindungsgemäßen Verfahrens (a) innerhalb eines größeren Bereiches variiert werden.

Im allgemeinen arbeitet man bei Temperaturen zwischen 0°C und 120°C, vorzugsweise zwischen 20°C und 100°C.

Bei der Durchführung der zweiten Stufe des erfindungsgemäßen Verfahrens (a) setzt man auf 1 Mol an Triazolyl-Derivat der Formel (Ia) im allgemeinen 1 bis 2 Mol an Halogen-Verbindung der Formel (III) sowie eine äquivalente Menge oder auch einen Überschuß an Säurebindemittel ein. Die Aufarbeitung erfolgt nach üblichen Methoden. Im allgemeinen geht man so vor, daß man das Reaktionsgemisch mit wäßriger Base und einem mit Wasser wenig mischbaren organischen Lösungsmittel versetzt, die organische Phase abtrennt, trocknet und einengt. Das erhaltene Produkt kann gegebenenfalls nach üblichen Methoden, z.B. durch Umkristallisation, von noch vorhandenen Verunreinigungen befreit werden.

Die bei der Durchführung der dritten Stufe des erfindungsgemäßen Verfahrens (a) als Ausgangssubstanzen benötigten Verbindungen der Formel (Ib) sind erfindungsgemäße Stoffe.

Als Oxidationsmittel kommen bei der Durchführung der dritten Stufe des erfindungsgemäßen Verfahrens (a) alle zur Oxidation von Schwefel üblichen Substanzen in Frage. Vorzugsweise verwendbar sind Wasserstoffperoxid und Persäuren, wie Peressigsäure und meta-Chlor-perbenzoesäure, und außerdem anorganische Salze, wie Kaliumpermanganat.

Als Verdünnungsmittel kommen bei der Durchführung der dritten Stufe des erfindungsgemäßen Verfahrens (a) alle für derartige Umsetzungen üblichen Solventien in Betracht. Verwendet man Wasserstoffperoxid oder Persäuren als Oxidationsmittel, so setzt man vorzugsweise Essigsäure oder Eisessig als Verdünnungsmittel ein. Arbeitet man mit Kaliumpermanganat als Oxidationsmittel, so kommen vorzugsweise Wasser oder Alkohole, wie tert.-Butanol, als Solventien in Frage.

Die Reaktionstemperaturen können bei der Durchführung der dritten Stufe des erfindungsgemäßen Verfahrens (a) innerhalb eines bestimmten Bereiches variiert werden. Im allgemeinen arbeitet man bei Temperaturen zwischen 0°C und 100°C, vorzugsweise zwischen 10°C und 100°C.

Bei der Durchführung der dritten Stufe des erfindungsgemäßen Verfahrens (a) setzt man auf 1 Mol an Verbindung der Formel (Ib) im allgemeinen eine äquivalente Menge oder einen Überschuß an Oxidationsmittel ein. Ist die Herstellung von SO-Verbindungen gewünscht, so arbeitet man im allgemeinen mit äquimolaren Mengen. Ist die Synthese von SO₂-Verbindungen beabsichtigt, so wählt man einen Überschuß an Oxidationsmitteln. Die Aufarbeitung erfolgt nach üblichen Methoden. Im allgemeinen geht man so vor, daß man mit Eis oder Wasser verdünnt, gegebenenfalls durch Zugabe von Base alkalisch stellt, mit einem mit Wasser wenig mischbaren, organischen Solvens extrahiert, die vereinigten organischen Phasen trocknet und einengt und das entstehende Produkt gegebenenfalls umkristallisiert. Arbeitet man mit Kaliumpermanganat in wäßriger Lösung, so verfährt man im allgemeinen in der Weise, daß man den Feststoff abfiltriert, wäscht und trocknet.

Die bei der Durchführung der ersten Stufe des erfindungsgemäßen Verfahrens (b) als Reaktionskomponenten benötigten Diaryldisulfide sind durch die Formel (IV) allgemein definiert.

Die Diaryl-disulfide der Formel (IV) sind bekannt oder lassen sich nach bekannten Methoden herstellen.

Als starke Basen kommen bei der Durchführung der ersten Stufe des erfindungsgemäßen Verfahrens (b) alle diejenigen starken Basen in Betracht, die bereits im Zusammenhang mit der Beschreibung der ersten Stufe des erfindungsgemäßen Verfahrens (a) genannt wurden.

Als Verdünnungsmittel kommen bei der Durchführung der ersten Stufe des erfindungsgemäßen Verfahrens (b) alle diejenigen Solventien in Betracht, die schon im Zusammenhang mit der Beschreibung der ersten Stufe des erfindungsgemäßen Verfahrens (a) genannt wurden.

Auch die übrigen Reaktionsbedingungen und die Aufarbeitungsmethoden bei der Durchführung der ersten Stufe des erfindungsgemäßen Verfahrens (b) entsprechen denjenigen, die bereits im Zusammenhang mit der Beschreibung der ersten Stufe des erfindungsgemäßen Verfahrens (a) genannt wurden.

Bei der Durchführung der zweiten Stufe des erfindungsgemäßen Verfahrens (b) kommen als Oxidationsmittel alle diejenigen Oxidantien in Betracht, die schon im Zusammenhang mit der Beschreibung der dritten Stufe des erfindungsgemäßen Verfahrens (a) genannt wurden.

Auch die Reaktionsbedingungen und die Aufarbeitungsmethoden sind bei der Durchführung der zweiten Stufe des erfindungsgemäßen Verfahrens (b) analog zu denjenigen, die schon im Zusammenhang mit der Beschreibung der dritten Stufe des erfindungsgemäßen Verfahrens (a) genannt wurden. Gleiches gilt für die Durchführung des erfindungsgemäßen Verfahrens (c).

Die nach den erfindungsgemäßen Verfahren erhältlichen Triazolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung im Pflanzenschutz als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen beispielsweise die folgenden Stoffe infrage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
lQuinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden.

Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

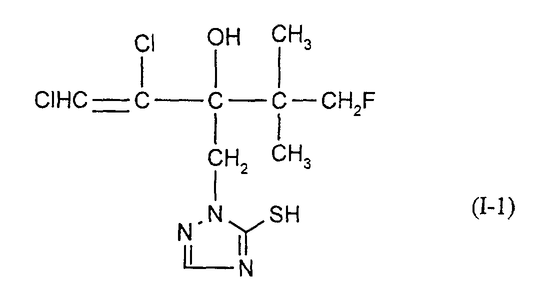

Ein Gemisch aus 1,41 g (5 mMol) 1,2-Dichlor-4,4-dimethyl-5-fluor-3-hydroxy-3-[(1,2,4-triazol-1-yl)-methyl]-1-penten und 25 ml absolutem Tetrahydrofuran wird bei - -70°C mit 4 ml (10 mMol) n-Butyl-lithium in Hexan versetzt und eine Stunde bei -70°C gerührt. Danach wird das Reaktionsgemisch mit 0,19 g (6 mMol) Schwefel-Pulver versetzt und 4 Stunden bei -70°C gerührt. Anschließend wird hydrolysiert, indem man 1 ml Methanol und 1 ml Essigsäure bei -70°C hinzufügt. Das Reaktionsgemisch wird zunächst mit Essigsäureethylester verdünnt und dann mehrfach mit gesättigter, wäßriger Ammoniumchlorid-Lösung ausgeschüttelt. Die organische Phase wird über Natriumsulfat getrocknet und unter vermindertem Druck eingeengt. Das anfallende Rohprodukt (1,7 g) wird durch Chromatographie an Kieselgel mit einem Gemisch aus Petrolether und Essigsäureethylester = 1:1 als Laufmittel gereinigt. Man erhält auf diese Weise 0,5 g (32 % der Theorie) an 1,2-Dichlor-4,4-dimethyl-5-fluor-3-hydroxy-3-[(5-mercapto-1,2,4-triazol-1-yl)-methyl]-1-penten in Form einer Festsubstanz vom Schmelzpunkt 162-164°C.

Nach den zuvor angegebenen Methoden werden auch die in der folgenden Tabelle 2 aufgeführten Stoffe hergestellt.

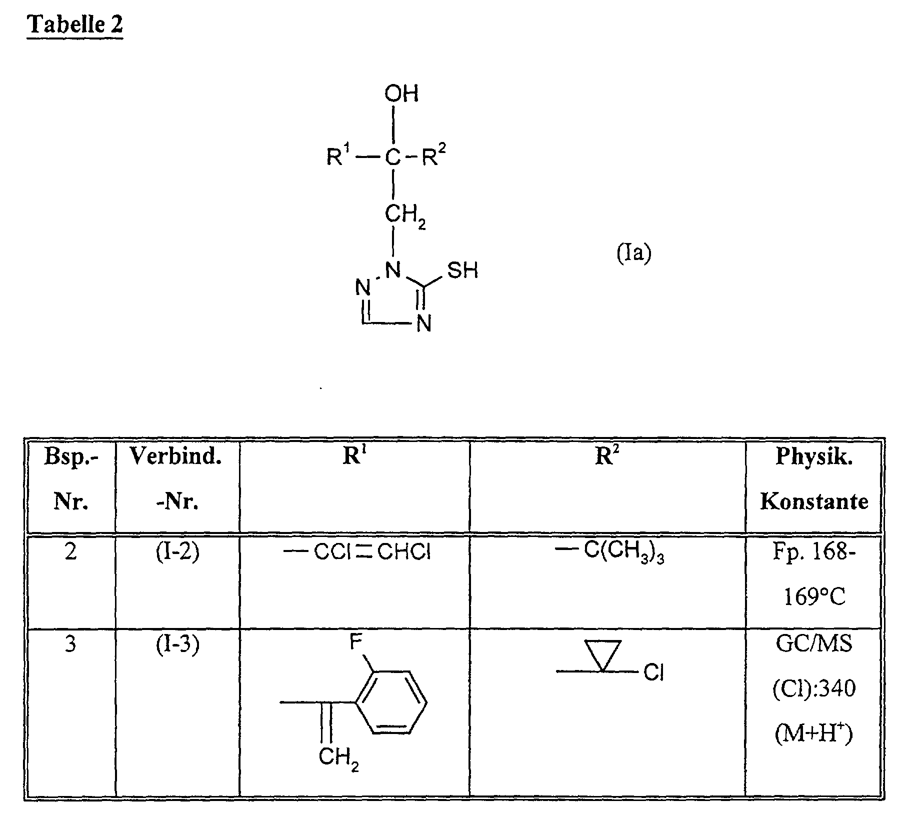

## Patentansprüche

1. Triazolyl-Derivate der Formel 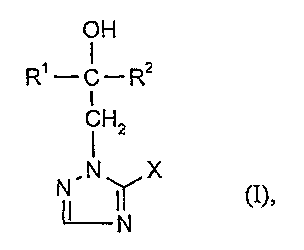 in welcher
R¹ für geradkettiges oder verzweigtes Alkenyl mit 2 bis 5 Kohlenstoffatomen steht, wobei jeder dieser Reste einfach bis dreifach, gleichartig oder verschieden substituiert sein kann durch Chlor, Brom, Methoxy, Ethoxy, Propoxy, Isopropoxy, Cyclopropyl, Cyclobutyl, Cyclopentyl und/oder Cyclohexyl,
und
R² für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluorcyclopropyl, 1-Methyl-cyclopropyl, 1-Cyano-Cyclopropyl, Cyclopropyl, 1-Methyl-cyclopentyl oder 1-Ethyl-cyclopentyl steht,
oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
R¹ für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
R² für 1-Methyl-cyclohexyl, Cyclohexyl, 1-Chlor-cyclopropyl, 1-Fluorcyclopropyl, 1-Methyl-cyclopropyl, 1-Cyano-Cyclopropyl, Cyclopropyl, 1-Methyl-cyclopentyl oder 1-Ethyl-cyclopentyl steht,
und
X für die Gruppierungen -SH, -SR³, -SO-R³, -SO₂-R³ oder -SO₃H steht, worin
R³ für Methyl, Ethyl oder Propyl steht, wobei jeder dieser Reste einfach bis dreifach, gleichartig oder verschieden durch Fluor und/oder Chlor substituiert sein kann,
oder
für Allyl, But-2-en-yl oder But-3-enyl steht, wobei jeder dieser Reste einfach bis dreifach, gleichartig oder verschieden substituiert sein kann durch Fluor und/oder Chlor,
oder
für Phenylalkyl mit 1 oder 2 Kohlenstoffatomen im geradkettigen oder verzweigten Alkylteil steht, wobei jeder dieser Reste im Phenylteil einfach oder zweifach substituiert sein kann durch Fluor, Chlor, Brom, Methyl, Ethyl, Tert.-Butyl, Trichlormethyl und/oder Trifluormethyl,
oder
für Phenyl steht, das einfach oder zweifach substituiert sein kann durch Fluor, Chlor, Brom, Methyl, Ethyl, tert.-Butyl, Trichlormethyl und/oder Trifluormethyl,
sowie deren Säureadditions-Salze und Metallsalz-Komplexen.

2. Verfahren zur Herstellung von Triazolyl-Derivaten der Formel (I) gemäß Anspruch 1
sowie von deren Säureadditions-Salzen und Metallsalz-Komplexen, **dadurch gekennzeichnet, daß** man
a) Hydroxyethyl-triazole der Formel 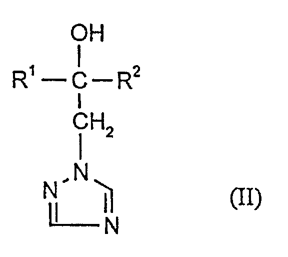 in welcher
R¹ und R² die in Anspruch 1 Bedeutungen haben,
entweder
α) nacheinander mit starken Basen und Schwefel in Gegenwart eines Verdünnungsmittels umsetzt und dann mit Wasser, gegebenenfalls in Gegenwart einer Säure hydrolysiert,
oder
β) mit Schwefel in Gegenwart eines hoch siedenden Verdünnungsmittels umsetzt und dann gegebenenfalls mit Wasser sowie gegebenenfalls mit Säure behandelt,
und gegebenenfalls die nach den Varianten (α) und (β) entstehenden Verbindungen der Formel 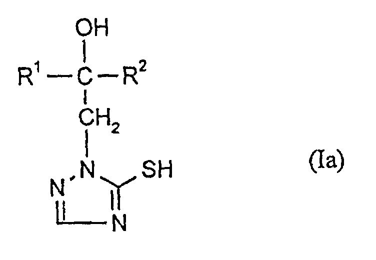 in welcher
R¹ und R² die oben angegebenen Bedeutungen haben,
mit Halogen-Verbindungen der Formel
R⁴-Hal (III)
in welcher
R⁴ für Methyl, Ethyl oder Propyl steht, wobei jeder dieser Reste einfach bis dreifach, gleichartig oder verschieden durch Fluor und/oder Chlor substituiert sein kann
oder
für Allyl, But-2-en-yl oder But-3-enyl steht, wobei jeder dieser Reste einfach bis dreifach, gleichartig oder verschieden substituiert sein kann durch Fluor und/oder Chlor,
oder
für Phenylalkyl mit 1 oder 2 Kohlenstoffatomen im geradkettigen oder verzweigten Alkylteil steht, wobei jeder dieser Reste im Phenylteil einfach oder zweifach substituiert sein kann durch Fluor, Chlor, Brom, Methyl, Ethyl, tert.-Butyl, Trichlormethyl und/oder Trifluormethyl,
und
Hal für Chlor, Brom oder Iod steht,
in Gegenwart eines Säurebindemittels und in Gegenwart eines Verdünnungsmittels umsetzt und gegebenenfalls die dabei entstehenden Verbindungen der Formel 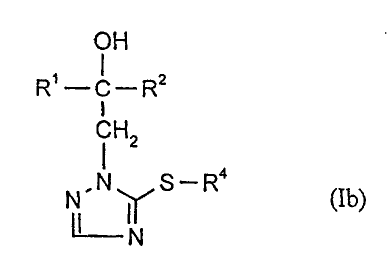 in welcher
R¹, R² und R⁴ die oben angegebenen Bedeutungen haben,
mit Oxidationsmitteln in Gegenwart eines Verdünnungsmittels umsetzt,
oder
b) Hydroxyethyl-triazole der Formel 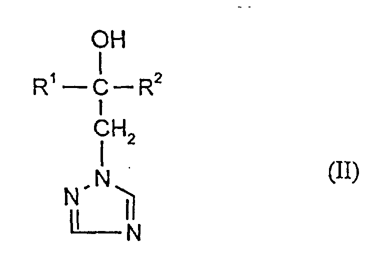 in welcher
R¹ und R² die oben angegebenen Bedeutungen haben,
nacheinander mit starken Basen und Diaryl-disulfiden der Formel
R⁵-S-S-R⁵ (IV)
in welcher
R⁵ für Phenyl steht, das einfach oder zweifach substituiert sein kann durch Fluor, Chlor, Brom, Methyl, ethyl, tert.-Butyl, Trichlormethyl und/oder Trifluormethyl,
in Gegenwart eines Verdünnungsmittels umsetzt und gegebenenfalls die dabei entstehenden Verbindungen der Formel 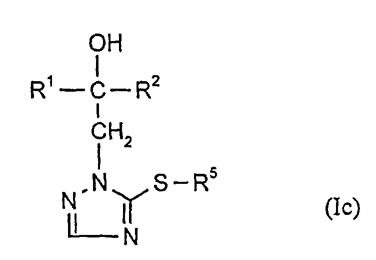 in welcher
R¹, R² und R⁵ die oben angegebenen Bedeutungen haben,
mit Oxidationsmitteln in Gegenwart eines Verdünnungsmittels umsetzt,
oder
c) Triazolyl-Derivate der Formel 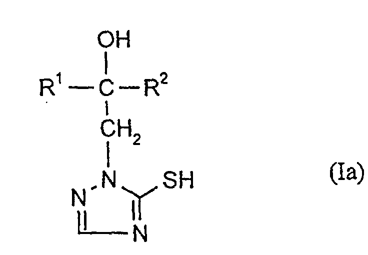 in welcher
R¹ und R² die oben angegebenen Bedeutungen haben,
mit Kaliumpermanganat in Gegenwart eines Verdünnungsmittels umsetzt,
und gegebenenfalls anschließend an die erhaltenen Verbindungen der Formel (I) eine Säure oder ein Metallsalz addiert.

3. Mikrobizide Mittel, **gekennzeichnet durch** einen Gehalt an mindestens einem Triazolyl-Derivat der Formel (I) gemäß Anspruch 1 bzw. an einem Säureadditions-Salz oder Metallsalz-Komplex eines Triazolyl-Derivates der Formel (I) neben Streckmitteln und / oder oberflächenaktiven Stoffen.

4. Verwendung von Triazolyl-Derivaten der Formel (I) gemäß Anspruch 1 sowie von deren Säureadditions-Salzen und Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, daß** man Triazolyl-Derivate der Formel (I) gemäß Anspruch 1 bzw. deren Säureadditions-Salze oder Metallsalz-Komplexe auf die Mikroorganismen und/oder deren Lebensraum ausbringt.

6. Verfahren zur Herstellung von mikrobiziden Mitteln, **dadurch gekennzeichnet, daß** man Triazolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

1. Triazolyl derivatives of the formula 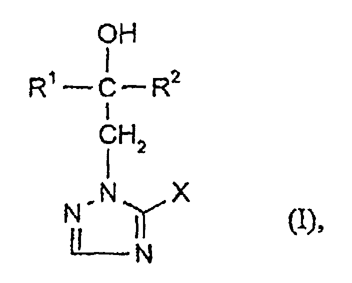 in which
R¹ represents straight-chain or branched alkenyl having 2 to 5 carbon atoms, it being possible for each of these radicals to be monosubstituted to trisubstituted by identical or different substituents from the series consisting of chlorine, bromine, methoxy, ethoxy, propoxy, isopropoxy, cyclopropyl, cyclobutyl, cyclopentyl and/or cyclohexyl
and
R² represents n-propyl, isopropyl, n-butyl, i-butyl, sec-butyl or tert-butyl, it being possible for these radicals to be monosubstituted to tetrasubstituted by identical or different substituents from the series consisting of fluorine, chlorine, bromine, methoxy, ethoxy, propoxy, isopropoxy, methoximino, ethoximino, cyclopropyl, cyclobutyl, cyclopentyl and/or cyclohexyl,
or
represents 1-methylcyclohexyl, cyclohexyl, 1-chlorocyclopropyl, 1-fluorocyclopropyl, 1-methylcyclopropyl, 1-cyanocyclopropyl, cyclopropyl, 1-methylcyclopentyl or 1-ethylcyclopentyl,
or
represents phenylalkyl having 1 or 2 carbon atoms in the straight-chain or branched alkyl moiety, it being possible for the phenyl moiety to be monosubstituted to trisubstituted by identical or different substituents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and/or cyano,
or
represents phenylalkenyl having 2 to 4 carbon atoms in the alkenyl moiety, it being possible for the phenyl moiety to be mono-substituted to trisubstituted by identical or different substituents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and/or cyano,
or
represents phenoxyalkyl having 1 to 4 carbon atoms in the straight-chain or branched oxyalkyl moiety, it being possible for the phenyl moiety to be monosubstituted to trisubstituted by identical or different substituents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and/or cyano,
or
represents phenyl which can be monosubstituted to trisubstituted by identical or different substituents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and/or cyano,
or
R¹ represents phenylalkyl having 2 to 4 carbon atoms in the alkenyl moiety, it being possible for the phenyl moiety to be monosubstituted to trisubstituted by identical or different substitutents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and/or cyano,
and
R² represents 1-methylcyclohexyl, cyclohexyl, 1-chlorocyclopropyl, 1-fluorocyclopropyl, 1-methylcyclopropyl, 1-cyanocyclopropyl, cyclopropyl, 1-methylcyclopentyl or 1-ethylcyclopentyl,
and
X represents the groups -SH, -SR³, -SO-R³, -SO₂R³ or -SO₃H, where
R³ represents methyl, ethyl or propyl, it being possible for each of these radicals to be monosubstituted to trisubstituted by identical or different substituents from the series consisting of fluorine and/or chlorine,
or represents
allyl, but-2-enyl or but-3-enyl, it being possible for each of these radicals to be monosubstituted to trisubstituted by identical or different substituents from the series consisting of fluorine and/or chlorine,
or represents
phenylalkyl having 1 or 2 carbon atoms in the straight-chain or branched alkyl moiety, it being possible for each of these radicals to be monosubstituted or disubstituted in the phenyl moiety by fluorine, chlorine, bromine, methyl, ethyl, tert-butyl, trichloromethyl and/or trifluoromethyl,
or represents
phenyl which can be monosubstituted or disubstituted by fluorine, chlorine, bromine, methyl, ethyl, tert-butyl, trichloromethyl and/or trifluoromethyl,
and their acid addition salts and metal salt complexes.

2. Process for the preparation of triazolyl derivatives of the formula (I) according to Claim 1
and of their acid addition salts and metal salt complexes, **characterized in that**
a) hydroxyethyltriazoles of the formula 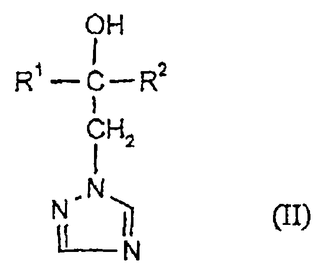 in which
R¹ and R² have the meanings stated in Claim 1
are either
α) reacted successively with strong bases and sulphur in the presence of a diluent and then hydrolysed with water, if appropriate in the presence of an acid,
or
β) reacted with sulphur in the presence of a high-boiling diluent and then, if appropriate, treated with water and, if appropriate, with acid,
and, if appropriate, the compounds of the formula 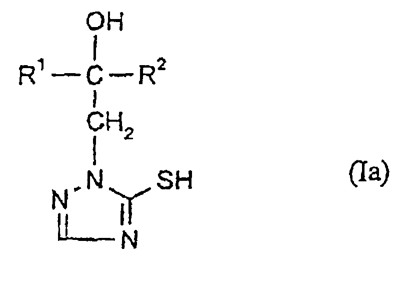 in which
R¹ and R² have the abovementioned meanings
which are obtained in accordance with variants (α) and (β)
are reacted with halogen compounds of the formula
R⁴-Hal (III)
in which
R⁴ represents methyl, ethyl or propyl, it being possible for each of these radicals to be monosubstituted to trisubstituted by identical or different substituents from the series consisting of fluorine and/or chlorine,
or represents
allyl, but-2-enyl or but-3-enyl, it being possible for each of these radicals to be monosubstituted to trisubstituted by identical or different substituents from the series consisting of fluorine and/or chlorine,
or represents
phenylalkyl having 1 or 2 carbon atoms in the straight-chain or branched alkyl moiety, it being possible for each of these radicals to be monosubstituted to disubstituted in the phenyl moiety by fluorine, chlorine, bromine, methyl, ethyl, tert-butyl, trichloromethyl and/or trifluoromethyl,
and
Hal represents chlorine, bromine or iodine,
if appropriate in the presence of an acid-binding agent and in the presence of a diluent and, if appropriate, the resulting compounds of the formula 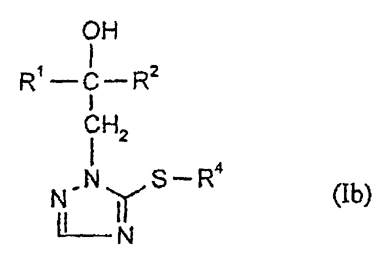 in which
R¹, R² and R⁴ have the abovementioned meanings
are reacted with oxidants in the presence of a diluent,
or
b) hydroxyethyltriazoles of the formula 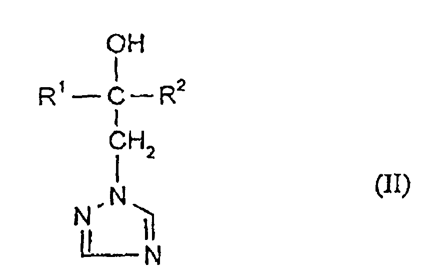 in which
R¹ and R² have the abovementioned meanings
are reacted successively with strong bases and diaryl disulphides of the formula
R⁵-S-S-R⁵ (IV)
in which
R⁵ represents phenyl which can be monosubstituted or disubstituted by fluorine, chlorine, bromine, methyl, ethyl, tert-butyl, trichloromethyl and/or trifluoromethyl
in the presence of a diluent and, if appropriate, the resulting compounds of the formula 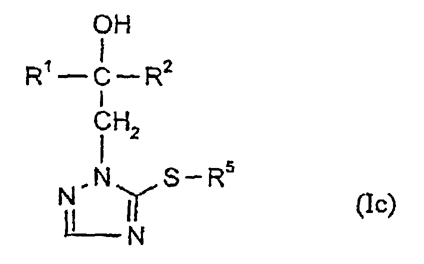 in which
R¹, R² and R⁵ have the abovementioned meanings
are reacted with oxidants in the presence of a diluent,
or
c) triazolyl derivatives of the formula 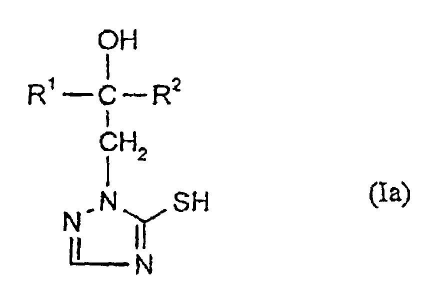 in which
R¹ and R² have the abovementioned meanings
are reacted with potassium permanganate in the presence of a diluent,
and, if appropriate, the resulting compounds of the formula (I) are then subjected to an addition reaction with an acid or a metal salt.

3. Microbicidal compositions, **characterized in that** they contain at least one triazolyl derivative of the formula (I) according to Claim 1 or an acid addition salt or metal salt complex of a triazolyl derivative of the formula (I), in addition to extenders and/or surface-active substances.

4. Use of triazolyl derivatives of the formula (I) according to Claim 1 and of their acid addition salts and metal salt complexes as microbicides in crop protection and in the protection of materials.

5. Method for controlling undesired microorganisms in crop protection and in the protection of materials, **characterized in that** triazolyl derivatives of the formula (I) according to Claim I or their acid addition salts or metal salt complexes are applied to the microorganisms and/or their environment.

6. Process for the preparation of microbicidal compositions, **characterized in that** triazolyl derivatives of the formula (I) according to Claim 1 or their acid addition salts or metal salt complexes are mixed with extenders and/or surface-active agents.

## Revendications

1. Dérivés de triazolyle de formule 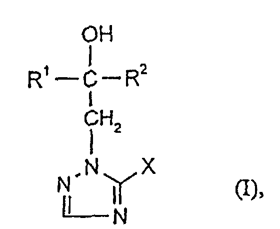 dans laquelle
R¹ est un reste alcényle linéaire ou ramifié ayant 2 à 5 atomes de carbone, qui peut être substitué dans chaque cas une à trois fois identiques ou différentes par du chlore, du brome, un radical méthoxy, éthoxy, propoxy, isopropoxy, cyclopropyle, cyclobutyle, cyclopentyle et/ou cyclohexyle,
et
R² est un reste n-propyle, isopropyle, n-butyle, iso-butyle, sec. -butyle ou tertio-butyle, qui peut être substitué une à quatre fois identiques ou différentes par du fluor, du chlore, du brome, un radical méthoxy, éthoxy, propoxy, isopropoxy, méthoximino, éthoximino, cyclopropyle, cyclobutyle, cyclopentyle et/ou cyclohexyle,
ou bien
un reste 1-méthylcyclohexyle, cyclohexyle, 1-chlorocyclopropyle, 1-fluorocyclopropyle, 1-méthylcyclopropyle, 1-cyanocyclopropyle, cyclopropyle, 1-méthylcyclopentyle ou 1-éthylcyclopentyle,
ou bien
un reste phénylalkyle ayant 1 ou 2 atomes de carbone dans la partie alkyle linéaire ou ramifiée, la partie phényle pouvant être substituée une à trois fois identiques ou différentes par un radical fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phénylalcényle ayant 2 à 4 atomes de carbone dans la partie alcényle, dont la partie phényle peut être substituée une à trois fois identiques ou différentes par un radical fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phénoxyalkyle ayant 1 à 4 atomes de carbone dans la partie oxyalkyle linéaire ou ramifiée et dont la partie phényle peut être substituée une à trois fois identiques ou différentes par un radical fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximino-éthyle, nitro et/ou cyano,
ou bien
un reste phényle qui peut être substitué une à trois fois identiques ou différentes par un radical fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximino-éthyle, nitro et/ou cyano,
ou bien
R¹ est un reste phénylalcényle ayant 2 à 4 atomes de carbone dans la partie alcényle, et dont la partie phényle peut être substituée une à trois fois identiques ou différentes par un radical fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluoro-méthoxy, chlorodifluorométhylthio, méthoxycarbonyle, éthoxycarbonyle, méthoximinométhyle, 1-méthoximinoéthyle, nitro et/ou cyano,
et
R² est un reste 1-méthylcyclohexyle, cyclohexyle, 1-chlorocyclopropyle, 1-fluorocyclopropyle, 1-méthylcyclopropyle, 1-cyanocyclopropyle, cyclopropyle, 1-méthylcyclopentyle ou 1-éthylcyclopentyle,
et
X représente les groupements -SH, -SR³, -SO-R³, -SO₂-R³ ou -SO₃H,
oû
R³ est un reste méthyle, éthyle ou propyle, chacun de ces restes pouvant être substitué une à trois fois identiques ou différentes par du fluor et/ou du chlore,
ou bien
un reste allyle, but-2-ényle ou but-3-ényle, chacun de ces restes pouvant être substitué une à trois fois identiques ou différentes par du fluor et/ou du chlore,
ou bien
un reste phénylalkyle ayant 1 ou 2 atomes de carbone dans la partie alkyle linéaire ou ramifiée, ce reste pouvant dans chaque cas être substitué une ou deux fois dans la partie phényle par du fluor, du chlore, du brome, un radical méthyle, éthyle, tertio-butyle, trichlorométhyle et/ou trifluorométhyle,
ou bien
un reste phényle qui peut être substitué une ou deux fois par du fluor, du chlore, du brome, un radical méthyle, éthyle, tertio-butyle, trichlorométhyle et/ou trifluorométhyle.
ainsi que leurs sels d'addition d'acides et leurs complexes de sels métalliques.

2. Procédé de production de dérivés de triazolyle de formule (I) suivant la revendication 1, ainsi que de leurs sels d'addition d'acides et de leurs complexes de sels métalliques, **caractérisé en ce que** :
a) des hydroxyéthyltriazoles de formule 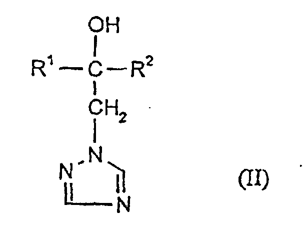 dans laquelle
R¹ et R² ont les définitions indiquées dans la revendication 1,
α) sont amenés à réagir successivement avec des bases fortes et du soufre en présence d'un diluant, puis hydrolysés avec de l'eau, éventuellement en présence d'un acide,
ou bien
β) amenés à réagir avec du soufre en présence d'un diluant de haut point d'ébullition, puis traités éventuellement avec de l'eau ainsi que, le cas échéant, avec un acide,
et les composés produits selon les variantes (α) et (β), de formule 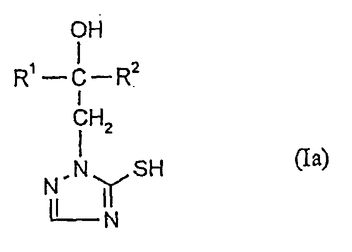 dans laquelle
R¹ et R² ont les définitions indiquées ci-dessus, sont amenés à réagir avec des composés halogénés de formule
R⁴-Hal (III)
dans laquelle
R⁴ représente un reste méthyle, éthyle ou propyle dont chacun peut être substitué une à trois fois identiques ou différentes par du fluor et/ou du chlore,
ou bien
un reste allyle, but-2-ényle ou but-3-ényle dont chacun peut être substitué une à trois fois identiques ou différentes par du fluor et/ou du chlore,
ou bien
un reste phénylalkyle ayant 1 ou 2 atomes de carbone dans la partie alkyle linéaire ou ramifiée, dont chacun peut être substitué dans la partie phényle une ou deux fois par du fluor, du chlore, du brome, un radical méthyle, éthyle, tertio-butyle, trichlorométhyle et/ou trifluorométhyle,
et
Hal représente le chlore, le brome ou l'iode,
en présence d'un accepteur d'acide et en présence d'un diluant et, le cas échéant, les composés ainsi produits de formule 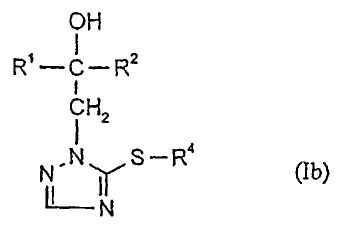 dans laquelle
R¹, R² et R⁴ ont les définitions indiquées ci-dessus,
sont amenés à réagir avec des agents oxydants en présence d'un diluant, ou bien
b) des hydroxyéthyltriazoles de formule 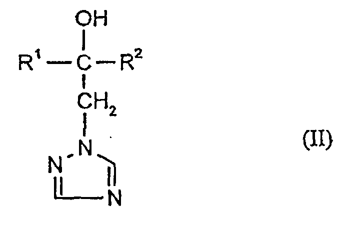 dans laquelle
R¹ et R² ont les définitions indiquées ci-dessus,
sont amenés à réagir successivement avec des bases fortes et des sulfures de diaryle de formule
R⁵-S-S-R⁵ (IV)
dans laquelle
R⁵ est un reste phényle qui peut être substitué une ou deux fois par du fluor, du chlore, du brome, un radical méthyle, éthyle, tertio-butyle, trichlorométhyle et/ou trifluorométhyle,
en présence d'un diluant et, le cas échéant, les composés ainsi produits de formule 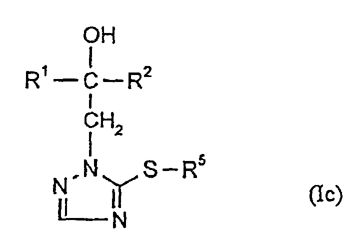 dans laquelle
R¹, R² et R⁵ ont les définitions indiquées ci-dessus,
sont amenés à réagir avec des agents oxydants en présence d'un diluant,
ou bien
c) des dérivés de triazolyle de formule 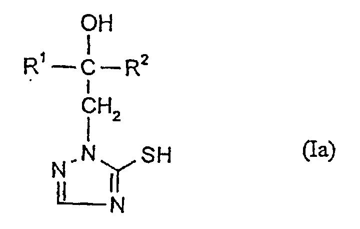 dans laquelle
R¹ et R² ont les définitions indiquées ci-dessus,
sont amenés à réagir avec du permanganate de potassium en présence d'un diluant,
puis, le cas échéant, un acide ou un sel métallique est additionné sur les composés de formule (I) obtenus.

3. Compositions microbicides, **caractérisées par** une teneur en au moins un dérivé de triazolyle de formule (I) suivant la revendication 1, ou un sel d'addition d'acide ou un complexe de sel métallique d'un dérivé de triazolyle de formule (I), à côté de diluants et/ou d'agents tensio-actifs.

4. Utilisation de dérivés de triazolyle de formule (I) suivant la revendication 1 ainsi que de leurs sels d'addition d'acides et de leurs complexes de sels métalliques comme microbicides dans la protection des plantes et dans la protection des matériaux.

5. Procédé de lutte contre des micro-organismes indésirables dans la protection des plantes et dans la protection des matériaux, **caractérisé en ce qu'**on fait agir des dérivés de triazolyle de formule (I) suivant la revendication 1 ou leurs sels d'addition d'acides ou leurs complexes de sels métalliques sur les micro-organismes et/ou sur leur milieu.

6. Procédé de préparation de compositions microbicides, **caractérisé en ce qu'**on mélange des dérivés de triazolyle de formule (I) suivant la revendication 1 ou leurs sels d'addition d'acides ou leurs complexes de sels métalliques avec des diluants et/ou des agents tensio-actifs.
